# EUROPÄISCHE PATENTSCHRIFT

(11) **EP 3 177 380 B1**
(45) Veröffentlichungstag und Bekanntmachung des Hinweises auf die Patenterteilung: **20.11.2019**
(21) Anmeldenummer: 15747792.8
(22) Anmeldetag: 04.08.2015
(51) Int. Cl.: B01D 3/32

(54) **KOLONNE ZUR THERMISCHEN BEHANDLUNG VON FLUIDEN GEMISCHEN MIT EINER TRAGEKONSTRUKTION ZWISCHEN ZWEI BÖDEN**
COLUMN FOR THERMALLY TREATING FLUID MIXTURES, HAVING A SUPPORT STRUCTURE BETWEEN TWO TRAYS
COLONNE DE TRAITEMENT THERMIQUE DE MÉLANGES FLUIDES PRÉSENTANT UNE STRUCTURE PORTEUSE ENTRE DEUX PLAQUES

(30) Priorität: 05.08.2014 DE 102014215438; 05.08.2014 US 201462033173 P
(43) Veröffentlichungstag der Anmeldung: 14.06.2017
(73) Patentinhaber: BASF SE, 67056 Ludwigshafen am Rhein (DE)
(72) Erfinder: HAMMON, Ulrich, 68163 Mannheim (DE); WALTER, Thomas, 67454 Haßloch (DE)
(74) Vertreter: BASF IP Association
(86) Internationale Anmeldenummer: PCT/EP2015/067912
(87) Internationale Veröffentlichungsnummer: WO 2016/020366

(56) Entgegenhaltungen:
- EP-A1- 2 108 421
- DE-A1- 19 530 291
- FR-A1- 2 940 137
- US-A- 3 632 315
- US-A1- 2012 111 717
- US-A1- 2012 228 251
- US-B1- 6 585 238

## Beschreibung

Die vorliegende Erfindung betrifft eine Kolonne zur thermischen Behandlung von fluiden Gemischen. Sie weist einen zylindrischen, vertikal ausgerichteten Kolonnenkörper auf, der einen Kolonnenhohlraum bildet. Die Kolonne umfasst ferner mehrere Böden, die vertikal beabstandet voneinander in dem Kolonnenhohlraum montiert sind. Des Weiteren weist die Kolonne eine Tragekonstruktion auf, die zumindest einen der Böden in vertikaler Richtung stützt. Bei der Kolonne handelt es sich insbesondere um eine Trennkolonne. Des Weiteren betrifft die Erfindung eine Bodeneinrichtung für eine solche Kolonne sowie ein thermisches Trennverfahren zwischen wenigstens einem in einer Kolonne aufsteigenden Gas und wenigstens einer in der Kolonne absteigenden Flüssigkeit.

In Trennkolonnen werden vielfach gasförmige (aufsteigend) und flüssige (absteigend) Stoffströme im Gegenstrom geführt, wobei wenigstens einer der Stoffströme insbesondere ein (Meth)acrylmonomer enthält. Infolge der zwischen den Stoffströmen bestehenden Ungleichgewichte findet ein Wärme- und Stoffaustausch statt, der letztlich die in der Trennkolonne gewünschte Abtrennung (bzw. Auftrennung) bedingt. In dieser Schrift sollen solche Trennverfahren als thermische Trennverfahren bezeichnet werden.

Beispiele für und damit Element der in dieser Schrift verwendeten Ausdrucksweise "thermische Trennverfahren" sind die fraktionierende Kondensation (vgl. z.B. DE 19924532 A1, DE 10243625 A1 und WO 2008/090190 A1) und die Rektifikation (bei beiden wird aufsteigende Dampfphase im Gegenstrom zu absteigender Flüssigphase geführt; die Trennwirkung beruht darauf, dass die Dampfzusammensetzung im Gleichgewicht von der Flüssigzusammensetzung verschieden ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit) und die Desorption (der Umkehrprozess zur Absorption; das in der Flüssigphase gelöste Gas wird durch Partialdruckerniedrigung abgetrennt; erfolgt die Partialdruckerniedrigung des in der Flüssigphase Gelösten wenigstens teilweise dadurch, dass ein Trägergas durch die Flüssigphase geleitet wird, bezeichnet man dieses thermische Trennverfahren auch als Strippung; alternativ oder auch zusätzlich (zeitgleich als Kombination) kann die Partialdruckerniedrigung durch eine Absenkung des Arbeitsdruckes bewirkt werden).

Beispielsweise kann die Abtrennung von (Meth)acrylsäure bzw. (Meth)acrolein aus dem Produktgasgemisch der katalytischen Gasphasenoxidation so durchgeführt werden, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Absorbat bzw. Kondensat nachfolgend unter Erhalt von mehr oder weniger reiner (Meth)acrylsäure bzw. (Meth)acrolein weiter aufgetrennt wird (vgl. z.B. DE-10332758 A1, DE 10243625 A1, WO 2008/090190 A1, DE 10336386 A1, DE 19924532 A1, DE 19924533 A1, DE 102010001228 A1, WO 2004/035514 A1, EP 1125912 A2, EP 982289 A2, EP 982287 A1 und DE 10218419 A1).

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure".

Im Besonderen sollen die in dieser Schrift angesprochenen (Meth)acrylmonomere die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, isoButylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe oder als Wasser super absorbierende Materialien in Hygieneartikeln Verwendung finden.

Großtechnisch werden (Meth)acrolein und (Meth)acrylsäure vorwiegend durch katalytische Gasphasenoxidation geeigneter C₃-/C₄-Vorläuferverbindungen (oder von Vorläuferverbindungen derselben) hergestellt. Im Fall von Acrolein und Acrylsäure werden als solche Vorläuferverbindungen bevorzugt Propen und Propan verwendet. Im Fall der Methacrylsäure und des Methacroleins sind iso-Buten und iso-Butan die bevorzugten Vorläuferverbindungen.

Neben Propen, Propan, iso-Buten und iso-Butan eignen sich als Ausgangsstoffe jedoch auch andere 3 bzw. 4 Kohlenstoffatome enthaltende Verbindungen wie z.B. iso-Butanol, n-Propanol oder Vorläuferverbindungen derselben wie z.B. der Methylether von iso-Butanol. Acrylsäure kann auch durch gasphasenkatalytische Oxidation von Acrolein erzeugt werden. Methacrylsäure kann auch durch gasphasenkatalytische Oxidation von Methacrolein erzeugt werden.

Im Rahmen solcher Herstellverfahren werden normalerweise Produktgemische erhalten, aus welchen die (Meth)acrylsäure bzw. das (Meth)acrolein abgetrennt werden muss.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgemische an, aus denen die (Meth)acrylsäureester abgetrennt werden müssen.

Die Trennkolonnen, in denen diese Trennverfahren durchgeführt werden, enthalten trennwirksame Einbauten. Diese verfolgen bei den thermischen Trennverfahren den Zweck, die Oberfläche für den die Auftrennung in der Trennkolonne bewirkenden Wärme- und Stoffaustausch ("die Austauschfläche") zu erhöhen.

Als solche Einbauten kommen z.B. Packungen, Füllkörper und/oder Böden, die auch als Stoffaustauschböden bezeichnet werden, in Betracht. Häufig werden als Trennkolonnen solche verwendet, die wenigstens als einen Teil der trennwirksamen Einbauten wenigstens eine Abfolge von Stoffaustauschböden enthalten.

Stoffaustauschböden verfolgen den Zweck, in der Trennkolonne in Form von auf ihnen sich ausbildenden Flüssigkeitsschichten Gebiete mit im Wesentlichen geschlossenen flüssigen Phasen zur Verfügung zu stellen. Die Oberfläche des in der Flüssigkeitsschicht aufsteigenden und sich dabei in der flüssigen Phase verteilenden Dampf- bzw. Gasstroms ist dann die maßgebliche Austauschfläche.

Unter einer Abfolge von Stoffaustauschböden wird dabei eine Aufeinanderfolge (ein Nacheinander) von wenigstens zwei in der Trennkolonne übereinander angeordneten, im Regelfall baugleichen (d.h., identischen), Stoffaustauschböden verstanden. Anwendungstechnisch vorteilhaft ist der lichte Abstand zwischen zwei in einer solchen Serie (Reihe) von Stoffaustauschböden unmittelbar aufeinanderfolgenden Stoffaustauschböden einheitlich gestaltet (d.h., die Stoffaustauschböden sind in der Trennkolonne äquidistant übereinander angeordnet).

Die einfachste Ausführungsform eines Stoffaustauschbodens ist der sogenannte Regensiebboden. Dabei handelt es sich um eine Platte bzw. um zu einer Platte zusammengefügte Plattensegmente, die für die aufsteigende Gas- bzw. Dampfphase (die Begriffe "gasförmig" und "dampfförmig" werden in dieser Schrift synonym verwendet) über die Platte verteilte und im Wesentlichen plane Durchtrittsöffnungen, z.B. runde Löcher und/oder Schlitze, aufweist (vgl. z.B. DE 10230219 A1, EP 1279429 A1, US-A 3988213 und EP 1029573 A1). Darüber hinausgehende Öffnungen (z.B. wenigstens einen Ablaufschacht (wenigstens ein Ablaufsegment)) weisen Regensiebböden nicht auf. Durch diese Abwesenheit von Ablaufschächten müssen sich sowohl das in der Trennkolonne aufsteigende Gas (der in der Trennkolonne aufsteigende Dampf) als auch die in der Trennkolonne absteigende Flüssigkeit entgegengesetzt strömend im zeitlichen Wechsel durch die (gleichen) Durchtrittsöffnungen (durch die offenen Querschnitte der Durchtrittstellen) bewegen. Man spricht auch vom "dual-flow" von aufsteigendem Gas und absteigender Flüssigkeit durch die Durchtrittsöffnungen, weshalb in der Literatur für solche Stoffaustauschböden häufig auch der Begriff "Dual-Flow-Böden" verwendet wird.

Der Querschnitt der Durchtrittsöffnungen eines Dual-Flow-Bodens wird in an sich bekannter Weise seiner Belastung angepasst. Ist er zu klein, strömt das aufsteigende Gas mit so hoher Geschwindigkeit durch die Durchtrittsöffnungen, dass die in der Trennkolonne absteigende Flüssigkeit im Wesentlichen ohne Trennwirkung mitgerissen wird. Ist der Querschnitt der Durchtrittsöffnungen zu groß, bewegen sich aufsteigendes Gas und absteigende Flüssigkeit im Wesentlichen ohne Austausch aneinander vorbei und der Stoffaustauschboden läuft Gefahr trocken zu laufen.

D.h., der trennwirksame Arbeitsbereich eines Regensiebbodens (Dual-Flow-Boden) weist zwei Grenzen auf. Eine minimale Grenzgeschwindigkeit des aufsteigenden Gases muss gegeben sein, damit auf dem Regensiebboden eine gewisse Flüssigkeitsschicht gehalten wird, um ein trennwirksames Arbeiten des Regensiebbodens zu ermöglichen. Die obere Grenze der Geschwindigkeit des aufsteigenden Gases ist durch den Flutpunkt festgelegt, wenn die Gasgeschwindigkeit zum Stau der Flüssigkeit auf dem Regensiebboden führt und ihr Durchregnen verhindert wird.

Die Längstausdehnung der Durchtrittsöffnungen eines technischen Dual-Flow-Bodens (= längste direkte Verbindungslinie zweier auf der Umrisslinie des Querschnitts der Durchtrittsöffnung liegender Punkte) beträgt in typischer Weise 10 bis 80 mm (vgl. z.B. DE 10156988 A1). Normalerweise sind die Durchtrittsöffnungen innerhalb eines Regensiebbodens identisch (d.h., sie weisen alle die gleiche geometrische Form und den gleichen Querschnitt (die gleiche Querschnittsfläche) auf). Anwendungstechnisch zweckmäßig handelt es sich bei ihren Querschnittsflächen um Kreise. D.h., bevorzugte Durchtrittsöffnungen von Regensiebböden sind kreisförmige Bohrungen. Die Relativanordnung der Durchtrittsöffnungen eines Regensiebbodens folgt vorteilhaft einer strengen Dreiecksteilung (vgl. z.B. DE 10230219 A1). Selbstverständlich können die Durchtrittsöffnungen innerhalb ein und desselben Regensiebbodens auch unterschiedlich gestaltet sein (über den Regensiebboden variieren).

Anwendungstechnisch vorteilhaft umfasst eine Abfolge von Regensiebböden in einer Trennkolonne baugleiche (identische) Regensiebböden, die vorzugsweise äquidistant übereinander angeordnet sind.

Gemäß der DE 10156988 A1 können aber auch Abfolgen von Regensiebböden in Trennkolonnen zur Anwendung kommen, deren Querschnitt (bevorzugt kreisförmig) innerhalb eines Dual-Flow-Bodens zwar einheitlich gestaltet ist, innerhalb der Abfolge jedoch variiert (z.B. von unten nach oben abnimmt).

In der Regel schließt jeder Dual-Flow-Boden einer entsprechenden Bodenabfolge mit der Wand der Trennkolonne bündig ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weist ein Regensiebboden üblicherweise allenfalls noch Öffnungen auf, die der Befestigung des Bodens auf Auflageringen oder ähnlichem dienen (vgl. z.B. DE 10159823 A1).

Im normalen Arbeitsbereich einer Abfolge von Regensiebböden regnet die in der Trennkolonne absteigende Flüssigkeit in Tropfen von Dual-Flow-Boden zu Dual-Flow-Boden, d.h., die zwischen den Dual-Flow-Böden aufsteigende Gasphase wird von einer zerteilten Flüssigkeitsphase durchsetzt. Die auf dem jeweils unteren Regensiebboden auftreffenden Tropfen werden beim Auftreffen teilweise versprüht. Der durch die Durchtrittsöffnungen strömende Gasstrom sprudelt durch die auf der Oberfläche des Bodens gebildete Flüssigkeitsschicht, wobei ein intensiver Stoff- und Wärmeaustausch zwischen der Flüssigkeit und dem Gas stattfindet.

Je nach Flüssigkeits- und Gasbelastung neigen Regensiebböden bei Kolonnendurchmesser von >2 m dazu, dass sich geringe Ungleichverteilungen von Flüssigkeiten aufschaukeln können und so der Flüssigkeitsholdup eines Bodens großflächig schwankt bzw. sich eine umlaufende Welle ausbilden kann, was zum einen die mechanische Stabilität des Kolonnenkörpers negativ beeinflussen kann und zum anderen die Trennwirkung vermindert, da die Flüssigkeitsverteilung unter diesen Bedingungen dann zeitlich und örtlich stark unterschiedlich ist. Zur Vermeiden solcher Instationaritäten hat es sich deshalb als vorteilhaft erwiesen Strömungsbrecher in Form von senkrecht stehenden Blechen auf dem Bodenquerschnitt zu verteilen, die ein Aufschaukeln der Flüssigkeit innerhalb des Kolonnenkörpers verhindern oder zumindest stark reduzieren. Die Höhe der Bleche sollte dabei in etwa der Höhe der sich ausbildenden Flüssigkeitssprudelschicht entsprechen. Diese beträgt bei üblichen Belastungen ca. 20 cm.

Der Querschnitt einer Trennkolonne ist in der Regel kreisförmig. Dies trifft in entsprechender Weise auf die zugehörigen Stoffaustauschböden zu.

Für die Zwecke dieser Schrift verwendbare Dual-Flow-Böden sind z.B. in Technische Fortschrittsberichte, Bd. 61, Grundlagen der Dimensionierung von Kolonnenböden, Seite 198 bis 211, Verlag Theodor Steinkopf, Dresden (1967) und in der DE 10230219 A1 beschrieben.

Von der vorstehend beschriebenen Abfolge von Regensiebböden, die Stoffaustauschböden ohne Zwangsführung der auf den Boden absteigenden Flüssigkeit auf dem Boden umfasst, werden Abfolgen von Stoffaustauschböden mit einer solchen Flüssigkeitszwangsführung unterschieden.

Diese Stoffaustauschböden sind dadurch gekennzeichnet, dass sie neben den bereits beschriebenen Durchtrittsöffnungen zusätzlich wenigstens einen Ablaufschacht aufweisen. Dabei handelt es sich um wenigstens eine im Stoffaustauschboden befindliche Ablauföffnung, der die auf den Stoffaustauschboden abgestiegene Flüssigkeit (z.B. über ein Ablaufwehr (dieses kann in einfachster Ausführungsform eine Verlängerung der Ablauföffnung mit einem Hals (einem Kamin; im Fall einer kreisförmigen Ablauföffnung einer Röhre) nach oben sein)) zufließt, und die in einen zum in der Abfolge darunter liegenden Stoffaustauschboden zulaufenden Schacht ausläuft, der in der Regel zu einer in Kolonnenlängsrichtung weisenden Achse zentralsymmetrisch ausgebildet ist. Der Querschnitt des Schachts kann entlang dieser Achse variieren (sich z.B. verjüngen) oder auch konstant sein.

Durch den wenigstens einen Ablaufschacht des Stoffaustauschbodens kann innerhalb einer Abfolge derartiger Stoffaustauschböden die von einem höher gelegenen Stoffaustauschboden absteigende Flüssigkeit unabhängig vom nach wie vor durch die Durchtrittsöffnungen dieses Stoffaustauschbodens aufsteigenden Gas bzw. Dampf als wenigstens ein Zulauf an Flüssigkeit auf den nächst tiefer gelegenen Stoffaustauschboden der Abfolge absteigen.

Wesentliche Grundlage für diese Auftrennung der Strömungswege von absteigender Flüssigkeit und aufsteigendem Gas ist der hydraulische Verschluss (der Flüssigkeitsverschluss oder auch Schachtverschluss) des jeweiligen Ablaufschachtes für das aufsteigende Gas (ein Ablaufschacht darf für das aufsteigende Gas keinen Bypass an den Durchtrittsöffnungen vorbei bilden; der Gasstrom (der Dampfstrom) darf nicht durch einen Ablaufschacht an den Durchtrittsöffnungen vorbei aufsteigen).

Ein solcher hydraulischer Verschluss kann z.B. dadurch erreicht werden, dass man den Ablaufschacht so weit nach unten zieht (so weit nach unten auslaufen lässt), dass er tief genug in die auf dem nächst tiefer gelegenen Stoffaustauschboden der Abfolge befindliche Flüssigkeitsschicht eintaucht (ein solcher Verschluss wird in dieser Schrift auch als "statischer Verschluss" bezeichnet). Der hierfür notwendige Flüssigkeitsstand kann auf dem tiefer gelegenen Stoffaustauschboden z.B. durch die Höhe entsprechender Ablaufwehre gewährleistet werden.

Eine derartige Ausführung hat jedoch den Nachteil, dass der Bereich des tiefer gelegenen Stoffaustauschbodens, der sich unmittelbar unterhalb des Auslaufquerschnitts eines Ablaufschachtes des darüber befindlichen Stoffaustauschbodens (die sogenannte Zulauffläche) befindet, keine Durchtrittsöffnungen für das aufsteigende Gas aufweisen kann und so nicht für den Stoff- und Wärmeaustausch zwischen der auf dem tiefer gelegenen Stoffaustauschboden ausgebildeten Flüssigkeitsschicht und dem aufsteigenden Gas zur Verfügung steht.

Bei einer alternativen Ausführungsform ist das untere Auslaufende des Ablaufschachtes so weit hochgezogen, dass es nicht mehr in die auf dem darunter liegenden Stoffaustauschboden befindliche Flüssigkeitsschicht eintaucht. In diesem Fall verbleibt zwischen dem unteren Ende des wenigstens einen Ablaufschachts und dem Stoffaustauschboden, auf den der Ablaufschacht zuläuft, ein ausreichend großer Zwischenraum, in dem sich eine Sprudelschicht ausbilden und ein Stoff- und Wärmeaustausch zwischen einer (auf dem unteren Stoffaustauschboden) auflaufenden Flüssigkeitsschicht und einem (durch diesen Boden) aufsteigenden Gas stattfinden kann. D.h., in diesem Fall kann auch die "Zulauffläche" des wenigstens einen Ablaufschachtes auf dem darunter befindlichen Stoffaustauschboden Durchtrittsöffnungen aufweisen und so die verfügbare Austauschfläche des Stoffaustauschbodens, und damit seine Trennwirkung vergrößert werden.

Ein statischer Flüssigkeitsverschluss des Ablaufschachtes kann in diesem Fall z.B. mit Hilfe einer unter dem Auslaufende des Ablaufschachtes angebrachten Auffangtasse bewirkt werden. Anwendungstechnisch zweckmäßig wird in diesem Fall die Mantelwand der Auffangtasse so weit hochgezogen, dass das Auslaufende des Ablaufschachts in die Auffangtasse eintaucht (es ist auch möglich, die Unterkante des Ablaufschachts an der Oberkante der Auffangtasse enden zu lassen). Beim Betrieb der Kolonne sammelt sich in der Auffangtasse die durch den Ablaufschacht herabströmende Flüssigkeit so lange, bis diese über die Oberkante der Mantelwand der Auffangtasse abfließt. Die Unterkante des Ablaufschachts taucht in die in der Auffangtasse befindliche Flüssigkeit ein und die Auffangtasse bildet einen siphonartigen Flüssigkeitsverschluss des Ablaufschachtes.

Alternativ kann ein hochgezogener Ablaufschacht auch dynamisch verschlossen werden. Hierzu kann der Ablaufschacht z.B. an seinem unteren Ende mit einem Boden verschlossen werden, der mit Austrittsöffnungen versehen ist, die so dimensioniert sind, dass die Flüssigkeit im Ablaufschacht aufgestaut und das Eindringen von Gas verhindert wird (vgl. z.B. EP 0882481 A1 und DE 10257915 A1). Der Schachtverschluss wird in diesem Fall dynamisch durch den Druckverlust, der an den Austrittsöffnungen entsteht, hergestellt. D.h., beim statischen Verschluss erfolgt der Verschluss des Ablaufschachtes dadurch, dass dessen Auslaufende in gestaute Flüssigkeit eintaucht, und beim dynamischen Verschluss bewirken konstruktive Merkmale am Auslaufende des Ablaufschachts, dass die austretende (auslaufende) Flüssigkeit einen Druckverlust erleidet, der im Ablaufschacht einen Rückstau der in selbigem absteigenden Flüssigkeit bewirkt, welcher den Verschluss bedingt. Im einfachsten Fall kann ein solcher Druckverlust dadurch verursacht werden, dass man den Querschnitt der Austrittsöffnung des Ablaufschachts im Vergleich zum mittleren Querschnitt des Schachts klein wählt.

Für ein trennwirksames Arbeiten einer Abfolge von derartigen Stoffaustauschböden ist die Ausführung des wenigstens einen Ablaufschachtes relevant. Einerseits muss der Querschnitt des wenigstens einen Ablaufschachts hinreichend groß gewählt werden (in der Regel ist die entsprechende Querschnittsfläche größer als die Querschnittsfläche einer Durchtrittsöffnung), damit die Flüssigkeit auch bei der maximalen Belastung der Trennkolonne mit selbiger noch sicher durch den wenigstens einen Ablaufschacht absteigen kann und nicht bis auf den darüber liegenden Boden zurückstaut. Auf der anderen Seite muss sichergestellt werden, dass auch bei minimaler Flüssigkeitsbelastung der hydraulische Verschluss des wenigstens einen Ablaufschachtes noch besteht.

Bei geringer Gasbelastung besteht ebenfalls die Gefahr eines Durchregnens von Flüssigkeit durch die Durchtrittsöffnungen. Darüber hinaus muss sich die Flüssigkeit in einem Ablaufschacht so weit aufstauen können, bis das Gewicht der gestauten Flüssigkeitssäule ausreicht, um die Flüssigkeit in den Gasraum unterhalb des zum Ablaufschachts gehörigen Stoffaustauschbodens zu befördern. Diese Rückstauhöhe bestimmt die erforderliche Mindestlänge des Ablaufschachts und bestimmt so den in einer Abfolge entsprechender Stoffaustauschböden erforderlichen Bodenabstand mit. Wesentlicher Mitbestimmungsfaktor für vorstehende Rückstauhöhe (Rückstaulänge) ist der Druckverlust ΔP eines Stoffaustauschbodens. Diesen Druckverlust erleidet das aufsteigende Gas beim Durchströmen der Durchtrittsöffnungen sowie der "hydrostatischen" Höhe der Sprudelschicht auf dem Stoffaustauschboden. Er ist dafür verantwortlich, dass der Druck in der Gasphase einer Abfolge solcher Stoffaustauschböden von oben nach unten zunimmt. Für den "hydrostatischen" Druck hₚ der im Ablaufschacht gestauten Flüssigkeit eines Stoffaustauschbodens muss daher wenigstens die Bedingung hₚ > ΔP des Stoffaustauschbodens erfüllt sein. Diese Zusammenhänge sind dem Fachmann z.B. aus der EP 1704906 A1 ebenso bekannt, wie die Möglichkeit, mit einem Zulaufwehr auf dem tiefer liegenden Stoffaustauschboden sicherzustellen, dass bei statischem Verschluss des Ablaufschachtes des oberhalb gelegenen Stoffaustauschbodens in der Flüssigkeitsschicht auf dem tiefer liegenden Stoffaustauschboden, der Schachtverschluss auch bei geringer Belastung mit absteigender Flüssigkeit noch besteht. Allerdings erhöht die Mitverwendung eines Zulaufwehrs die Rückstauhöhe, die im Ablaufschacht erforderlich ist, um die in selbigem gestaute Flüssigkeit auf den tiefer gelegenen Stoffaustauschboden zu drücken. Insgesamt ermöglicht das Element des Ablaufschachts eine Verbreiterung des trennwirksamen Arbeitsbereichs im Vergleich zum Regensiebboden. Eine günstige Ablaufgeschwindigkeit der im Ablaufschacht gestauten Flüssigkeit aus dem Ablaufschacht heraus beträgt beim erfindungsgemäßen Verfahren z.B. 1,2 m/s.

Zusätzlich ermöglicht es eine Zwangsführung der auf einen Stoffaustauschboden absteigenden Flüssigkeit auf diesem Boden.

Weist z. B. nur eine Hälfte eines (vorzugsweise kreisförmigen) Stoffaustauschbodens wenigstens einen Ablaufschacht auf (d.h., alle Ablauföffnungen befinden sich mit ihrem vollen Umfang innerhalb des entsprechenden Kreissegments) und sind in einer Abfolge von wenigstens zwei baugleichen derartigen Stoffaustauschböden die Stoffaustauschböden in einer Trennkolonne so übereinander angeordnet, dass zwei von oben nach unten aufeinanderfolgende Stoffaustauschböden in der Trennkolonne jeweils um 180° um die Kolonnenlängsachse gegeneinander verdreht (gedreht) angebracht sind, so dass sich ihre Ablaufschächte auf einander gegenüber liegenden Seiten (in einander gegenüberliegenden Hälften) der Trennkolonne befinden, so muss sich die von einem oberen Stoffaustauschboden durch seinen wenigstens einen Ablaufschacht auf den darunter angebrachten Stoffaustauschboden absteigende Flüssigkeit auf diesem unteren Stoffaustauschboden über den unteren Stoffaustauschboden betrachtet von der wenigstens einen Zulauffläche des wenigstens einen Ablaufschachts des oberen (des darüber angebrachten) Stoffaustauschbodens (von dem wenigstens einen Zulauf durch den wenigstens einen Ablaufschacht des oberen Stoffaustauschbodens) in notwendiger Weise (d. h., gezwungenermaßen) zu dem wenigstens einen Ablaufschacht dieses unteren Stoffaustauschbodens strömen. D. h., die vom oberen auf den unteren Boden absteigende Flüssigkeit wird zwangsweise quer über den Boden von dem wenigstens einen Zulauf zu dem wenigstens einen Ablauf geführt.

Eine solche Flüssigkeitsführung auf einem Stoffaustauschboden innerhalb einer Abfolge von baugleichen Stoffaustauschböden soll in dieser Schrift als eine Querströmung, die Abfolge von solchen baugleichen Stoffaustauschböden als eine Abfolge von baugleichen Querstrom-Stoffaustauschböden und der einzelne Stoffaustauschboden innerhalb der Abfolge als Querstrom-Stoffaustauschboden bezeichnet werden.

Im einfachsten Fall ist der Querstrom-Stoffaustauschboden ein Querstrom-Siebboden. Abgesehen von dem wenigstens einen Ablaufschacht weist er Durchtrittsöffnungen für das in einer Trennkolonne aufsteigende Gas auf, für deren Ausgestaltung grundsätzlich alle beim Regensiebboden angesprochenen Ausführungsformen in Betracht kommen. Vorzugsweise weist ein Querstrom-Siebboden als Durchtrittsöffnungen ebenfalls kreisförmige Bohrungen auf, die anwendungstechnisch vorteilhaft ebenfalls einen einheitlichen Radius aufweisen. Wie bereits erwähnt, ermöglicht es der wenigstens eine Ablaufschacht, dass die in einer Trennkolonne absteigende Flüssigkeit in einer Abfolge von Querstrom-Siebböden unabhängig vom Strömungsweg des in der Abfolge aufsteigenden Dampfes (durch die Durchtrittsöffnungen hindurch) von einem höher gelegenen Querstrom-Siebboden auf den nächst tiefer gelegenen Querstrom-Siebboden absteigen kann. Auf dem tiefer gelegenen Boden fließt die Flüssigkeit im Querstrom von dem durch den wenigstens einen Ablauf des höher gelegenen Querstrom-Siebbodens gebildeten wenigstens einen Zulauf des tiefer gelegenen Bodens zu dem wenigstens einen Ablaufschacht (zu dem wenigstens einen Ablauf) des tiefer gelegenen Bodens, wobei z.B. die Höhe von wenigstens einem Ablaufwehr, über das die Flüssigkeit dem wenigstens einen Ablaufschacht zufließen kann, die gewünschte Flüssigkeitshöhe auf dem tiefer gelegenen Querstrom-Siebboden mit gewährleistet. Zusätzlich wird die Flüssigkeit durch den Staudruck des in der Trennkolonne aufsteigenden Dampfes auf dem Querstrom-Siebboden gehalten. Sinkt die Dampfbelastung eines Querstrom-Siebbodens jedoch unter einen Mindestwert, kann es zum Durchregnen der Flüssigkeit durch die Durchtrittsöffnungen kommen, was die Trennwirkung des Querstrom-Siebbodens mindert und/oder zum Trockenlaufen des Querstrom-Siebbodens führt.

Dieser Gefahr des Trockenlaufens kann dadurch entgegengewirkt werden, dass die Ablauföffnung des wenigstens einen Ablaufschachts ablaufbewehrt ist und die jeweilige Durchtrittsöffnung mit einem Hals (einem Kamin; im Fall einer kreisförmigen Durchtrittsöffnung einer Röhre) nach oben verlängert wird.

Über dem Halsende sind normalerweise Dampfumlenkhauben (Glocken, umgedrehte Tassen) angebracht (diese können im einfachsten Fall mit dem Hals (z.B. vorne und hinten) verschraubt aufsitzen und werden praktisch über den Hals gestülpt), die in die auf dem Boden aufgestaute Flüssigkeit eintauchen. Der durch die jeweilige Durchtrittsöffnung aufsteigende Dampf strömt zunächst durch deren Hals in die zugehörige Haube, in welcher er umgeleitet wird, um anschließend, im Unterschied zum Querstrom-Siebboden, parallel zur Bodenfläche aus der Haube in die auf selbiger gestaute Flüssigkeit zu strömen (eine solche "Parallelausströmung" ist bei erfindungsgemäßen Verfahren in der Regel insofern günstig, als sie in unerwünschter Weise gebildete Polymerisatpartikel "wegzublasen" und dadurch einen Selbstreinigungseffekt zu bewirken vermag). Die aus benachbarten, über den Böden vorzugsweise äquidistant verteilt angeordneten, Hauben austretenden Gasströme (Dampfströme) wirbeln die auf dem Boden gestaute Flüssigkeit auf und bilden in selbiger eine Sprudelschicht aus, in der der Stoff- und Wärmeaustausch stattfindet. Solche Querstrom-Stoffaustauschböden werden auch als Querstrom-Glockenböden bzw. Querstrom-Haubenböden bezeichnet. Da sie auch bei geringer Belastung mit aufsteigendem Gas (Dampf) gestaute Flüssigkeit aufweisen und so keine Gefahr laufen, trocken zu laufen, werden sie auch als hydraulisch abgedichtete Querstromböden bezeichnet. Im Vergleich zu Querstrom-Siebböden bedürfen sie üblicherweise höherer Investitionskosten und bedingen höhere Druckverluste des durch sie hindurch aufsteigenden Gases. Die wie beschrieben ausgeführte (ausgestaltete) Durchtrittsöffnung dieser Böden wird im Unterschied zur einfachen Siebdurchtrittsöffnung eines Siebbodens auch als Glockendurchtrittsöffnung bzw. Haubendurchtrittsöffnung bezeichnet.

Das wichtigste Bauelement des Querstrom-Glockenbodens ist die Glocke (vgl. z. B. DE 10243625 A1 und Chemie-Ing.-Techn. 45. Jahrg. 1973/Nr. 9 + 10, S. 617 bis 620). Je nach Gestalt und Anordnung der Glocken (Dampfumlenkhauben, Hauben) unterscheidet man Querstrom-Glockenböden z. B. in Querstrom-Rundglockenböden (die Querschnitte von Durchtrittsöffnung, Kamin (Hals) und Glocke (Dampfumlenkhaube) sind rund (z. B. der Zylinderglockenboden oder der Flachglockenboden), Tunnel-Querstromböden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante parallel zur Querstromrichtung der Flüssigkeit ausgerichtet ist) und Querstrom-Thormann®böden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante senkrecht zur Querstromrichtung der Flüssigkeit ausgerichtet ist). Querstrom-Thormannböden sind z. B. in der DE 19924532 A1 und in der DE 10243625 A1 und dem in diesen beiden Schriften gewürdigten Stand der Technik beschrieben.

Der Glockenrand kann bei Querstrom-Glockenböden sehr verschiedene Formen aufweisen (vgl. DE 10243625 A1 und Chemie-Ing. Techn. 45. Jahrg. 1973/Nr. 9 + 10, S. 617 bis 620). Abbildung 3 aus Chemie-Ing. Techn. 45 Jahr. 1973/Nr. 9 + 10, S. 618 zeigt einige Beispiele für den gezackten und den geschlitzten Rand. Die Zacken und Schlitze sind üblicherweise so geformt, dass sich der aus der Glocke heraus in die auf dem Stoffaustauschboden aufgestaute Flüssigkeit eintretende Dampf möglichst leicht in eine große Zahl von Blasen oder Dampfstrahlen auflöst. Vorgenannte Abbildung 3 sowie verschiedene Figuren der DE 10243625 A1 zeigen außerdem beispielhafte Ausführungsformen von Glockenrändern, die eine sägezahnartige Struktur aufweisen, deren Zähne zusätzlich mit Leitflügeln (Leitflächen) ausgestattet sind ("aufgebogene Schlitze"). Die Leitflügel sollen dem aus den aufgebogenen sägezahnartigen Schlitzen austretenden Gasstrom (Dampfstrom) eine tangentiale Austrittsrichtung aufzwingen (den Gasaustritt in die Flüssigkeit in eine schräge Richtung leiten) wodurch die umgebende Flüssigkeit einen gerichteten Bewegungsimpuls erhält, was im Zusammenwirken mit der Anordnung der Glocken (Dampfumlenkhauben) zu einer gerichteten Flüssigkeitsströmung auf dem Querstrom-Glockenboden führen kann, die sich der über den Stoffaustauschboden betrachtet einstellenden Querströmung überlagert (häufig werden derartige aufgebogene Schlitze auch als Treibschlitze bezeichnet). Beispielsweise fließt in einer Abfolge von Querstrom-Thormannböden die Flüssigkeit auf einem tiefer gelegenen Querstrom-Thormannboden nicht auf direktem Weg quer über den Boden, sondern auf vorstehend beschriebene Weise angetrieben mäandrierend von dem wenigstens einen Zulauf zu dem wenigstens einen Ablauf. Der Raum zwischen zwei in Querstromrichtung hintereinander angeordneten Hauben eines Querstrom-Thormannbodens bildet jeweils eine Rinne, in der die Flüssigkeit fließt. Die Detailausgestaltung eines Querstrom-Thormannbodens erfolgt darüber hinaus normalerweise so, dass die Flüssigkeit in zwei in Querstromrichtung jeweils aufeinanderfolgenden Rinnen im Gegenstrom fließt (vgl. z. B. Figur 3 der DE 10243625 A1). Das auf diese Weise resultierende Mäandrieren der Querströmung verlängert den Strömungsweg der Flüssigkeit von dem wenigstens einen Zulauf zu dem wenigstens einen Ablauf, was die Trennwirkung eines Querstrom-Thormannbodens begünstigt.

Wie bereits ausgeführt, wird bei einem Querstrom-Glockenboden das aus der Glocke austretende Gas, im Unterschied zum Querstrom-Siebboden, parallel zur Bodenfläche in die auf dem Querstrom-Glockenboden aufgestaute Flüssigkeit eingeleitet. Reibungs- und Auftriebskräfte sorgen dann dafür, dass mit zunehmendem Abstand des ausgetretenen Gasstroms vom Glockenrand immer mehr Teilströme desselben in eine Richtung senkrecht zum Querstrom-Glockenboden umgelenkt werden und schließlich aus der Flüssigkeitsschicht ausdringen. Mit zunehmender Gasbelastung einer Glocke wächst die Geschwindigkeit des aus ihr austretenden Gasstroms, was den Abstand vom Rand der Glocke ("den Wirkungsbereich der Glocke"), bis zu welchem vorstehend beschriebene Umlenkung erfolgt ist, vergrößert.

Dieser Abhängigkeit des Wirkungsbereichs einer starren Glocke von der Gasbelastung kann dadurch entgegengewirkt werden, dass die Durchtrittsöffnung eines Querstrom-Stoffaustauschbodens als Ventil (als Ventildurchtrittsöffnung) ausgestaltet (ausgeführt) wird. Die dabei resultierenden Querstrom-Stoffaustauschböden werden als Querstrom-Ventilböden bezeichnet (vgl. z. B. DD 279822 A1, DD 216633 A1 und DE 102010001228 A1).

Der Begriff Querstrom-Ventilböden subsumiert in dieser Schrift somit Querstrom-Stoffaustauschböden, die Durchtrittsöffnungen (Bodenbohrungen) mit hubbegrenzten Teller-, Ballast- oder Hebeventilen (Schwimmklappen) aufweisen, die die Größe der Dampfdurchtrittsöffnung der jeweiligen Kolonnenbelastung anpassen.

In einer einfachen Ausgestaltung werden die Durchtrittsöffnungen des Bodens zu vorgenanntem Zweck mit nach oben beweglichen Deckeln oder Tellern (Scheiben) abgedeckt. Beim Durchtritt des aufsteigenden Gases werden die Deckel (Teller, Scheiben) durch den Gasstrom in einem über die jeweilige Durchtrittsöffnung zusätzlich angebrachten (das normalerweise am Boden fest verankert ist) entsprechenden Führgerüst (Führkäfig) angehoben und erreichen schließlich eine der Gasbelastung entsprechende Hubhöhe (anstelle eines Führkäfigs kann die Scheibe auch über mit dem Boden verankerte aufwärtsbewegliche Ventilbeine verfügen, deren Aufwärtsbeweglichkeit nach oben begrenzt ist). Der durch die Durchtrittsöffnung aufsteigende Gasstrom wird an der Unterseite des angehobenen Deckels (Tellers, Scheibe) in ähnlicher Weise wie in der Glocke (bei einer Glockendurchtrittsöffnung) umgelenkt und tritt aus dem unter dem angehobenen Teller (Deckel, Scheibe) entstandenen Austrittsbereich aus und wie beim Glockenboden parallel zum Boden in die auf selbigem aufgestaute Flüssigkeit ein. Der Tellerhub steuert so die Größe des Gasaustrittsbereichs und passt sich selbständig der Kolonnenbelastung an, bis das obere Ende des Führkäfigs die maximal mögliche Hubhöhe begrenzt. Die Teller können dabei nach unten gerichtete Distanzhalter aufweisen, sodass bei niedriger Gasbelastung das Ventil nur so weit schließt, dass der durch die Distanzhalter geschaffene Raum noch eine intensive Vermischung der horizontalen Gasausströmung mit der querströmenden Flüssigkeit gestattet. Distanzhalter wirken auch einem Anhaften der Ventilscheibe am Boden entgegen. Durch geeignete Ausgestaltung der Ventilelemente eines Querstrom-Ventilbodens kann die Blasrichtung des Ventilelements eingestellt und so die Flüssigkeitszwangsführung auf dem Querstrom-Ventilboden zusätzlich beeinflusst werden (vgl. z. B. DD 216 633 A1). Das Prinzip von Querstrom-Ventilböden sowie für die Zwecke der vorliegenden Schrift verwendbare Ventilböden findet sich z. B. in Technische Fortschrittsberichte, Band 61, Grundlagen der Dimensionierung von Kolonnenböden, Seite 96 bis 138 ausgeführt. Neben den vorstehend beschriebenen beweglichen Ventilen kennt der Fachmann auch noch feststehende Ventile. Dabei handelt es sich normalerweise um scheibenförmige, oder trapezförmige, oder rechteckige Einheiten, die aus der Bodenplatte herausgestanzt werden und mit dieser über nach oben gerichtete feststehende Beine verbunden sind.

Insbesondere bei größeren Durchmessern einer Trennkolonne bildet sich auf Querstrom-Stoffaustauschböden von dem wenigstens einen Zulauf ausgehend bis zum Erreichen des Ablaufwehrs des wenigstens einen Ablaufs in natürlicher Weise ein zu beachtendes Flüssigkeitsgefälle aus (der Gradient der Stauhöhe der Flüssigkeit speist (bedingt) die Querströmung). Dies hat zur Folge, dass in Bereichen mit einer geringeren Flüssigkeitshöhe aufgrund der daraus resultierenden geringeren Widerstände der aufsteigende Dampf (das aufsteigende Gas) vergleichsweise leichter durch die Flüssigkeitsschicht hindurchtreten kann. Daraus kann schließlich eine ungleichmäßige Gasbelastung des Querstrom-Stoffaustauschbodens erwachsen (die Bereiche mit einer geringeren Flüssigkeitshöhe (einem geringeren Durchströmungswiderstand) werden bevorzugt durchströmt), die die Trennwirkung desselben beeinträchtigt. Durch die Anwendung von z. B. in ihrer Sitzhöhe einstellbaren Glocken (alternativ kann auch die Glockengröße verändert werden) bei Querstrom-Glockenböden bzw. durch Verwendung von z. B. Tellern (Deckeln) mit unterschiedlichem Gewicht bei Querstrom-Ventilböden kann diesbezüglich ausgleichend eingewirkt werden, so dass der Stoffaustauschboden über seinen Querschnitt im Wesentlichen gleichmäßig gast (dort wo die Flüssigkeitshöhe auf dem Querstrom-Stoffaustauschboden kleiner ist, wird die Sitzhöhe der Glocke anwendungstechnisch zweckmäßig entsprechend tiefer liegend bzw. das Gewicht des Hubtellers (Hubdeckels) entsprechend höher liegend gewählt; die Sitzhöhe der Glocke kann z.B. auch dadurch tiefer gelegt werden, dass die Länge des entsprechenden Kamins, an dessen Ende die Glocke gegebenenfalls verschraubt aufsitzt, gezielt verkürzt wird; alternativ oder zusätzlich kann z.B. auch die Zacken-/ Schlitzstruktur des Glockenrands variiert werden, um den erwünschten Strömungswiderstandsausgleich zu bewirken; idealer Weise erfolgt die Einstellung über den Querstrom-Stoffaustauschboden so, dass im Betrieb der Trennkolonne jede der auf einem Querstrom-Glockenboden befindliche Glocke den gleichen Strömungswiderstand für das aufsteigende Gas bedingt). Im Übrigen sind die Durchtrittstellen (die Durchtrittsöffnungen) eines Querstrom-Stoffaustauschbodens in der Regel vorteilhaft einheitlich gestaltet.

Durch einen Querstrom-Stoffaustauschboden hindurch verlaufende Öffnungen (von oben nach unten), deren Querschnittsfläche üblicherweise mehr als 200 mal kleiner als die Gesamtquerschnittsfläche aller übrigen Öffnungen des Querstromstoffaustauschbodens (den Querschnitt des wenigstens einen Ablaufschachtes nicht miteinbezogen) ist, bilden keine (trennwirksamen) Durchtrittsöffnungen für das durch den Querstrom-Stoffaustauschboden aufsteigende Gas und werden selbigen daher nicht zugerechnet. Beispielsweise kann es sich bei solchen Öffnungen um winzige Leerlaufbohrungen handeln, über die hydraulisch abgedichtete Querstromböden beim Abschalten einer Trennkolonne leerlaufen können. Auch können solche Öffnungen Verschraubungszwecken dienen.

Abfolgen von wenigstens einen Ablaufschacht aufweisenden Stoffaustauschböden, bei denen sich der wenigstens eine Zulauf und der wenigstens eine Ablauf z. B. in der gleichen Hälfte des (kreisförmigen) Stoffaustauschbodens befinden oder bei denen sich der wenigstens eine Zulauf in Bodenmitte und der wenigstens eine Ablauf am Rand des Bodens befindet, bilden keine Abfolge von Querstrom-Stoffaustauschböden im anmeldegemäßen (erfindungsgemäßen) Sinn.

Der Wirkungsgrad von wie beschrieben ausgeführten Querstrom-Stoffaustauschböden liegt üblicherweise unterhalb desjenigen eines theoretischen Bodens (einer theoretischen Trennstufe). Als theoretischer Boden (oder theoretische Trennstufe) soll in dieser Schrift ganz generell diejenige Raumeinheit einer für ein thermisches Trennverfahren eingesetzten, trennwirksame Einbauten enthaltenden, Trennkolonne verstanden werden, die eine Stoffanreicherung entsprechend dem thermodynamischen Gleichgewicht bewirkt. D. h., der Begriff des theoretischen Bodens ist sowohl auf Trennkolonnen mit Stoffaustauschböden, als auch auf Trennkolonnen mit Packungen und/oder Füllkörpern anwendbar.

Der Stand der Technik empfiehlt den Einsatz von Abfolgen von wenigstens zwei baugleichen (identisch ausgeführten) Querstrom- Stoffaustauschböden u. a. in trennwirksame Einbauten enthaltenden Trennkolonnen, die zur Durchführung thermischer Trennverfahren zwischen wenigstens einem in der Trennkolonne aufsteigenden Gasstrom und wenigstens einem in der Trennkolonne absteigenden Flüssigkeitsstrom zur Anwendung kommen, und wobei wenigstens einer der Ströme wenigstens ein (Meth)acrylmonomeres enthält. Beispielsweise empfehlen die Schriften DE 19924532 A1, DE 10243625 A1 und WO 2008/090190 A1 die Mitverwendung einer Abfolge baugleicher hydraulisch abgedichteter Querstrom-Stoffaustauschböden in einer Trennkolonne zur Durchführung eines Verfahrens der fraktionierenden Kondensation eines Acrylsäure enthaltenden Produktgasgemischs einer heterogen katalysierten Gasphasen-Partialoxidation von C₃-Vorläufern der Acrylsäure mit molekularem Sauerstoff, die von unten nach oben zunächst Dual-Flow-Böden und im Anschluss daran hydraulisch abgedichtete Querstrom-Stoffaustauschböden enthält.

Charakteristisch für die Abfolgen der im Stand der Technik empfohlenen Querstrom-Stoffaustauschböden ist, dass der jeweils untere von zwei in der Abfolge aufeinanderfolgenden Querstrom-Stoffaustauschböden in Richtung der Querströmung von seinem wenigstens einen Zulauf zu seinem wenigstens einen Ablaufschacht nur im Bereich zwischen dem wenigstens einen Zulauf und dem wenigstens einen Ablaufschacht (der wenigstens einen Ablauföffnung) Durchtrittsöffnungen aufweist (vgl. z. B. die Figuren 3 und 4 der DE 10243625 A1, die Figur 1 der DD 279822 A1, die Figur 1 der DD 216633 A1 und die Abbildung 1 aus Chemie-Ing.-Techn. 45. Jahrgang, 1973/Nr. 9 + 10, Seite 617 bis 620).

Die Erfindung betrifft insbesondere Kolonnen, bei denen die vorgenannten Böden eingesetzt werden.

Häufig enthalten Trennkolonnen für thermische Trennverfahren Stützelemente als Tragekonstruktion für die Böden. Die WO 2004/092108 A1 beschreibt z. B. eine Trennkolonne, bei welcher der Stoffaustauschboden von einem Doppel-T-Träger unterstützt wird.

Bei Kolonnen mit einem sehr großen Innendurchmesser ergibt sich das Problem, dass die im Kolonnenhohlraum montierten Böden instabil werden. Um die Böden von Kolonnenkörpern zu stabilisieren, deren Durchmesser beispielsweise größer als 5 m ist, wurden Tragekonstruktionen eingesetzt wie z.B. in US2012/0228251A1 und US3632315, um die Böden in vertikaler Richtung zu stützen. Hierbei ergab sich jedoch das Problem, dass sich die thermische Behandlung von fluiden Gemischen in der Kolonne verschlechtert hat. Die Trennwirkung reduzierte sich dramatisch.

Der vorliegenden Erfindung lag daher die Aufgabe zugrunde, eine Kolonne der eingangs genannten Art bereitzustellen, bei der die volle Trennleistung auch dann gewährleistet ist, wenn der Kolonnenkörper einen großen Durchmesser aufweist. Des Weiteren soll eine Bodeneinrichtung für eine solche Kolonne sowie ein thermisches Trennverfahren zwischen wenigstens einem in einer Kolonne aufsteigenden Gas und wenigstens einer in der Kolonne absteigenden Flüssigkeit angegeben werden.

Erfindungsgemäß wird diese Aufgabe durch eine Kolonne mit den Merkmalen des Anspruchs 1, sowie ein Verfahren mit den Merkmalen des Anspruchs 10 gelöst. Vorteilhafte Ausgestaltungen und Weiterbildungen ergeben sich aus den abhängigen Ansprüchen.

Demgemäß wurde eine Kolonne zur thermischen Behandlung von fluiden Gemischen gefunden, die einen zylindrischen, vertikal ausgerichteten Kolonnenkörper, der einen Kolonnenhohlraum bildet, mehrere Böden, die vertikal beabstandet voneinander in dem Kolonnenhohlraum montiert sind, und eine Tragekonstruktion, die zumindest einen der Böden in vertikaler Richtung stützt, aufweist. Die erfindungsgemäße Kolonne ist dadurch gekennzeichnet, dass die Tragekonstruktion mehrere Öffnungen aufweist, die einen horizontalen Stoffaustausch durch die Tragekonstruktion erlauben.

Unter einer Tragekonstruktion wird in dieser Schrift verstanden, dass durch die Tragekonstruktion zumindest ein Boden der Kolonne vertikal abgestützt wird. Die Tragekonstruktion ist somit so in der Kolonne montiert, dass sie diese Stützfunktion erfüllen kann. Ferner sind die Materialstärken der Tragekonstruktion so ausgelegt, dass die Stützfunktion erfüllt werden kann. Unter einer Tragekonstruktion werden somit insbesondere nicht so genannte Wellenbrecher verstanden, die an einem Boden befestigt sind und die verhindern sollen, dass es zu einer wellenförmigen Anregung der auf einem Boden befindlichen Flüssigkeit kommt.

Die Böden der Kolonne sind insbesondere horizontal ausgerichtet und vertikal beabstandet voneinander in dem Kolonnenhohlraum montiert. Dabei wird z. B. eine nach unten ausgerichtete horizontale Fläche gebildet, welche von der Tragekonstruktion gestützt wird.

Bei sehr großen Kolonnendurchmessern müssen Träger eingesetzt werden, deren vertikale Ausdehnungen in den Bereich des Bodenabstandes kommen. Dadurch entstehen kaskadierte Bereiche, die nicht im Queraustausch stehen und bei denen Ungleichverteilungen durch Querströmungen nicht hinreichend ausgeglichen werden können. Im schlimmsten Fall können sich Bereiche bilden, in denen Flüssigkeit und Gas nahezu vollkommen getrennt geführt werden. Es wurde festgestellt, dass dieser Effekt zu der nachteiligen Reduzierung der Trennwirkung bei großen Kolonnendurchmessern führte.

Es wurde somit gefunden, dass die Verschlechterung der Trennleistung bei einem sehr großen Kolonnendurchmesser dadurch auftritt, dass die Tragekonstruktion einen Stoffaustausch bei einem Boden in horizontaler Richtung verhindert. Es ergibt sich in einem solchen Fall eine horizontale Kaskadierung durch die Tragekonstruktion zwischen zwei vertikal benachbarten Böden. Bei der erfindungsgemäßen Kolonne weist die Tragekonstruktion nun Öffnungen auf, die eine solche Kaskadierung verhindern, da sie einen horizontalen Stoffaustausch durch die Tragekonstruktion hindurch erlauben. Hierdurch wurde erreicht, dass der nachteilige Effekt der Reduktion der Trennleistung bei Bodenträgern, bei denen die Trägerhöhe im Bereich des Bodenabstands liegt, nicht mehr auftritt.

Bei einem vertikalen Schnitt durch die Tragekonstruktion nehmen die Öffnungen einen Anteil an der Schnittfläche der Tragekonstruktion ein, der in einem Bereich von 30% bis 90% liegt, bevorzugt in einem Bereich von 40% bis 80% liegt und besonders bevorzugt in einem Bereich von 50% bis 60% liegt. Diese Bereichsangaben für den Anteil der Öffnungen an der Schnittfläche der Tragekonstruktion gelten insbesondere für alle vertikalen Schnitte durch die Tragekonstruktion.

Es hat sich herausgestellt, dass insbesondere bei dem einleitend genannten Einsatz der Kolonne zur thermischen Behandlung, insbesondere Trennung, von fluiden Gemischen eine ausreichende Trennwirkung nur dann sichergestellt werden kann, wenn eine ausreichende Querströmung der Flüssigkeit auf dem Boden möglich ist. Bei vielen Verfahren ist es hierfür erforderlich, dass die Öffnungen einen Anteil an der vertikalen Schnittfläche der Tragekonstruktion von zumindest 40%, bevorzugt zumindest 50%, einnehmen. In Abhängigkeit von dem durchgeführten Trennverfahren tritt bei großen Kolonnendurchmessern von beispielsweise über 5 m eine erhebliche Verschlechterung der Trennwirkung auf, wenn die Öffnungen einen geringeren Anteil an der vertikalen Schnittfläche der Tragekonstruktion einnehmen.

Gemäß einer bevorzugten Ausgestaltung der Kolonne weist die Tragekonstruktion zumindest einen Träger auf. Die Öffnungen sind in diesem Fall in einer vertikal ausgerichteten Wandfläche des Trägers gebildet. Bei dem Träger kann es sich beispielsweise um einen Doppel-T-Träger, einen T-Träger, einen U-Träger oder einen Z-Träger handeln. Die Öffnungen sind dann in der jeweils vertikal ausgerichteten Wand des Trägers gebildet.

Bevorzugt weist die Tragekonstruktion mehrere Träger auf, wobei auch in diesem Fall die Öffnungen in vertikal ausgerichteten Wandflächen der Träger gebildet sind. Die Träger können beispielsweise in horizontaler Richtung beabstandet voneinander einen Boden in vertikaler Richtung stützen.

Bei einem Ausführungsbeispiel der erfindungsgemäßen Kolonne entspricht die Höhe des Trägers bzw. der Träger dem vertikalen Abstand benachbarter Böden, so dass der Träger bzw. die Träger zwischen benachbarten Böden angeordnet sind. Auf diese Weise wird auch bei sehr großen Kolonnendurchmessern eine stabile Tragekonstruktion für die Böden bereitgestellt.

Bei einem Ausführungsbeispiel der erfindungsgemäßen Kolonne wird zumindest ein Teil der Öffnungen nach unten nur von einem Steg begrenzt, der auf der Oberseite des Bodens aufliegt. Die Öffnungen reichen somit sehr weit bis zur Oberseite des Bodens heran. Auf diese Weise kann eine Flüssigkeit, die sich auf der Oberseite eines Bodens befindet, fast ungehindert durch den Träger hindurch in horizontaler Richtung fließen.

Die räumlichen Begriffe "oben", "unten", "horizontal" und "vertikal", beziehen sich, soweit nichts anderes ausdrücklich erwähnt ist, auf die Orientierung der Kolonne während des Betriebs.

Gemäß einer anderen Ausgestaltung der Kolonne ist die Tragekonstruktion ein Gittertragwerk oder ein Fachwerk. Ein Gittertragwerk umfasst dabei Träger, die aus einer Vielzahl von Stäben bestehen, die diagonal angeordnet und an den Kreuzungspunkten miteinander zu einem Gitter verbunden sind. Ein Fachwerk ist eine Konstruktion aus mehreren Stäben, die an beiden Enden miteinander verbunden sind. Jeder Stab ist dabei Bestandteil mindestens eines Fachs. Ein Fach ist somit ein Mehreck aus Stäben. Durch diese Ausbildung der Tragekonstruktion werden sehr große Öffnungen für einen besonders guten horizontalen Stoffaustausch bereitgestellt, wobei die Tragekonstruktion jedoch eine ausreichende Stabilität für die Böden selbst bei einem sehr großen Kolonnendurchmesser bereitstellt.

Die Tragekonstruktion liegt auf einem Boden auf und sie stützt einen darüber angeordneten Boden von unten. In diesem Fall ist es vorteilhafterweise nicht erforderlich, die Tragekonstruktion selbst im Kolonnenkörper zu befestigen. Die Tragekonstruktion stützt sich auf einen unteren im Kolonnenkörper befestigten Boden ab und stützt auf diese Weise den darüber liegenden Boden insbesondere im Bereich der Bodenmitte. Auch hierdurch kann eine besonders stabile Konstruktion für die Böden im Kolonnenkörper bereitgestellt werden.

Gemäß einer weiteren Ausgestaltung der erfindungsgemäßen Kolonne ist bei zumindest einer Teilmenge der Öffnungen die untere Begrenzungskante der Öffnung von dem Boden gebildet, auf dem die Tragekonstruktion aufliegt, so dass diese Teilmenge der Öffnungen einen ungehinderten horizontalen Stoffaustausch auf der Ebene dieses Bodens durch die Öffnungen erlaubt. Bei diesen Öffnungen ist die Tragekonstruktion somit nach unten hin offen, so dass Flüssigkeit auf dem Boden durch diese Öffnungen hindurch fließen kann. Hierdurch kann ein besonders guter horizontaler Flüssigkeitsaustausch auf dem Boden, auf dem die Tragekonstruktion aufliegt, bereitgestellt werden.

Alternativ wird die untere Begrenzungskante der Öffnung nicht von dem Boden selbst, sondern von einem Steg der Tragekonstruktion gebildet, der auf der Oberseite des Bodens aufliegt. Dieser Steg ist jedoch nur sehr schmal, so dass er den horizontalen Stoffaustausch auf der Ebene des Bodens nur unwesentlich beeinträchtigt.

Gemäß einer weiteren Ausgestaltung der Kolonne weist die Tragekonstruktion zumindest einen Träger auf. Dieser Träger umfasst einen oberen Horizontalschenkel, einen Vertikalschenkel und einen unteren Horizontalschenkel, wobei der obere Horizontalschenkel einen oberen Boden stützt, der Vertikalschenkel durch einen unteren Boden durchtritt und dieser von dem unteren Horizontalschenkel von unten gestützt wird.

Die Öffnungen der Tragekonstruktion sind bei dieser Ausgestaltung in dem Vertikalschenkel gebildet. Ein Teil der Öffnungen, insbesondere die unteren Öffnungen können dabei so in dem Vertikalschenkel angeordnet sein, dass die untere Kante der Öffnung auf der Höhe der oberen Oberfläche des unteren Bodens angeordnet ist. Durch diese Ausbildung der Tragekonstruktion wird ein sehr guter horizontaler Stoffaustausch bereitgestellt, wobei die Tragekonstruktion jedoch gleichzeitig eine sehr große Stabilität aufweist und zwei Böden stützen kann.

Gemäß einer Weiterbildung der erfindungsgemäßen Kolonne sind zumindest zwei Öffnungen vertikal beabstandet übereinander angeordnet. Dies bedeutet, dass zumindest bereichsweise sich die beiden übereinander angeordneten Öffnungen in vertikaler Richtung überlappen. Die Seitenbegrenzungen der übereinander angeordneten Öffnungen sind insbesondere in denselben Vertikalebenen angeordnet. Außerdem ist die Form dieser zwei übereinander angeordneten Öffnungen insbesondere identisch. Sie können gegebenenfalls spiegelsymmetrisch angeordnet sein. Die Öffnungen können beispielsweise eine Trapezform aufweisen. Durch diese Ausgestaltung wird vorteilhafterweise erreicht, dass der Stoffaustausch zwischen zwei Böden in verschiedenen Horizontalebenen gewährleistet ist, wobei gleichzeitig eine hohe Stabilität der Tragekonstruktion erhalten bleibt.

Gemäß der Ausgestaltung der Erfindung sind die Öffnungen in zumindest zwei horizontalen Reihen angeordnet, die vertikal beabstandet voneinander sind. Die Öffnungen der zwei horizontalen Reihen können insbesondere horizontal versetzt zueinander angeordnet sein. Auch hierdurch wird ein Stoffaustausch in verschiedenen Horizontalebenen ermöglicht, wobei gleichzeitig die Stabilität der Tragekonstruktion nicht beeinträchtigt wird.

Gemäß einer Ausgestaltung der erfindungsgemäßen Kolonne besitzen die Öffnungen eine Wabenstruktur. Beispielsweise ist zumindest ein Teil der Öffnungen sechseckig. Hierdurch kann eine besonders hohe Stabilität für die Böden bereitgestellt werden, wobei gleichzeitig durch die Wabenstruktur ein horizontaler Stoffaustausch auf einem Boden ermöglicht wird.

Bei den zwischen dem Träger oder den Trägern und den Böden gebildeten Bereichen können weitere trennwirksame Einbauten angeordnet sein. Durch die trennwirksamen Einbauten wird die Stofftrennung in einer Kolonne, die als Trennkolonne eingesetzt wird, verbessert.

Diese weiteren Einbauten können beispielsweise in Form von Packungen, insbesondere strukturierten bzw. geordneten Packungen, und/oder Schüttungen vorgesehen sein. Unter den Schüttungen sind solche mit Ringen, Wendeln, Sattelkörpern, Raschig-, Intos- oder Pall-Ringen, Berl- oder Intalox-Sätteln, Top-Pak etc. bevorzugt. Für erfindungsgemäß zu verwendende Extraktionskolonnen besonders geeignete Packungen sind z. B. Packungen der Julius Montz GmbH in D-40705 Hilden, wie z. B. die Packung Montz-Pak B1-350. Vorzugsweise verwendet man gelochte strukturierte Packungen aus Edelstahlblechen. Packungskolonnen mit geordneten Packungen sind dem Fachmann an sich bekannt und z. B. in Chem.-Ing.Tech. 58 (1986) Nr. 1, S. 19 - 31 sowie in der Technischen Rundschau Sulzer 2/1979, S. 49 ff. der Gebrüder Sulzer Aktiengesellschaft in CH-Winterthur beschrieben.

Die erfindungsgemäße Kolonne kann insbesondere als Trennkolonne eingesetzt werden. Die Trennkolonne weist eine Abfolge von Böden, insbesondere Stoffaustauschböden auf. Als Stoffaustauschböden werden insbesondere die eingangs genannten Böden verwendet, d. h. Stoffaustauschböden ohne Zwangsführung, wie Regensiebböden und Dual-Flow-Böden, und Stoffaustauschböden mit einer Flüssigkeitszwangsführung, wie z. B. Querstrom-Stoffaustauschböden, insbesondere Querstrom-Glockenböden, Querstrom-Haubenböden, Querstrom-Thormannböden und Querstrom-Ventilböden.

Der lichte Abstand zwischen zwei innerhalb der erfindungsgemäßen Kolonne unmittelbar aufeinanderfolgenden Böden beträgt insbesondere nicht mehr als 700 mm, vorzugsweise nicht mehr als 600 mm bzw. nicht mehr als 500 mm. Anwendungstechnisch zweckmäßig ist der lichte Abstand innerhalb der Bodenabfolge 300 bis 500 mm. Im Regelfall sollte der Bodenabstand 250 mm nicht unterschreiten.

Unter einem großen Durchmesser des Kolonnenkörpers wird in diese Schrift ein Durchmesser von zumindest 5 m verstanden. Der Kolonnenkörper der erfindungsgemäßen Kolonne weist insbesondere eine horizontale Maximalerstreckung auf, die zumindest 5 m ist, und insbesondere größer als 5 m, bevorzugt größer als 7 m ist. Falls der Kolonnenkörper einen kreisringförmigen Querschnitt besitzt, ist die horizontale Maximalerstreckung der Innendurchmesser des Kolonnenkörpers.

Die Höhe des Kolonnenkörpers ist beispielsweise größer als 5 m, insbesondere größer als 10 m. Es ist jedoch auch möglich, dass die Höhe des Kolonnenkörpers 30 m oder 40 m übersteigt.

Die Erfindung betrifft des Weiteren eine Bodeneinrichtung für eine Kolonne zur thermischen Behandlung von fluiden Gemischen mit einem Boden zur Anordnung in einem Kolonnenhohlraum einer Kolonne und einer Tragekonstruktion für den Boden, die mehrere Öffnungen aufweist, die einen zum Boden parallelen Stoffaustausch durch die Tragekonstruktion erlauben. Wenn der Boden der Bodeneinrichtung im Einsatz in der Kolonne z. B. horizontal ausgerichtet ist, ermöglichen die Öffnungen der Tragekonstruktion einen horizontalen Stoffaustausch durch die Tragekonstruktion hindurch. Bei einem Einsatz der Bodeneinrichtung in einer Kolonne können dieselben Vorteile erzielt werden, wie bei der vorstehend erläuterten Kolonne.

Die Erfindung betrifft des Weiteren ein thermisches Trennverfahren zwischen wenigstens einem in einer Kolonne, wie sie vorstehend beschrieben wurde, aufsteigenden Gas und wenigstens einer in der Kolonne absteigenden Flüssigkeit. Dabei enthält das aufsteigende Gas und/oder die absteigende Flüssigkeit insbesondere (Meth)acrylmonomere.

Es hat sich herausgestellt, dass bei einem thermischen Trennverfahren, bei dem das aufsteigende Gas oder die absteigende Flüssigkeit (Meth)acrylmonomere enthält, der Stoffaustausch in horizontaler Richtung besonders wichtig ist. Um eine ausreichende Trennwirkung zu gewährleisten, ist es in diesem Fall insbesondere erforderlich, dass die Öffnungen einen Anteil an der horizontalen Schnittfläche der Tragekonstruktion einnehmen, der größer als 40%, insbesondere größer als 50% ist.

Das erfindungsgemäße thermische Trennverfahren kann z.B. ein Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure sein.

Die Trennkolonne (Kondensationskolonne) kann wie in den Schriften DE 10243625 A1 bzw. WO 2008/090190 A1 beschrieben ausgeführt sein, sieht man davon ab, dass die dort eingesetzten Böden von der erfindungsgemäßen Bodeneinrichtung gebildet sind, d. h. die beschriebene Tragekonstruktion umfassen.

Im Folgenden werden Ausführungsbeispiele der erfindungsgemäßen Kolonne und Ausführungsbeispiele des erfindungsgemäßen Verfahrens mit Bezug zu den Zeichnungen erläutert.
- Figur 1: zeigt eine schematische Ansicht einer Kolonne gemäß einem Ausführungsbeispiel der Erfindung,
- Figur 2: zeigt einen Querschnitt der Kolonne des in Figur 1 gezeigten Ausführungsbeispiels,
- Figur 3: zeigt eine Teilansicht eines Vertikalschnitts der Kolonne gemäß dem in Figur 1 gezeigten Ausführungsbeispiel mit einem ersten Beispiel einer Tragekonstruktion,
- Figur 4: zeigt eine Teilansicht eines Vertikalschnitts der Kolonne gemäß dem in Figur 1 gezeigten Ausführungsbeispiel mit einem zweiten Beispiel einer Tragekonstruktion und
- Figur 5: zeigt eine Teilansicht eines Vertikalschnitts der Kolonne, der senkrecht zu dem in Figur 4 gezeigten Vertikalschnitt ist, gemäß dem in Figur 1 gezeigten Ausführungsbeispiel mit dem zweiten Beispiel der Tragekonstruktion.

Das im Folgenden beschriebene Ausführungsbeispiel betrifft eine Trennkolonne 1, wie sie z. B. bei einem Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure eingesetzt wird.

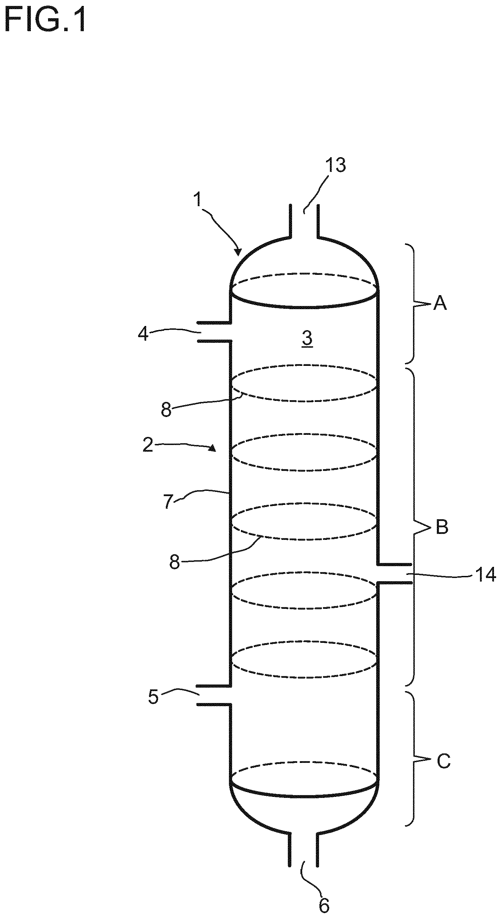

In Fig. 1 ist die an sich bekannte Trennkolonne 1 schematisch dargestellt. Sie umfasst einen zylindrischen Kolonnenkörper 2, dessen Achse vertikal ausgerichtet ist. Bei dem Kolonnenkörper 2 handelt es sich im Wesentlichen um einen Hohlzylinder. Das heißt, der Mantel 7 des Kolonnenkörpers 2 bildet einen Kolonnenhohlraum 3. Der Kolonnenkörper 2 ist aus Edelstahl gefertigt. Nach außen ist die Trennkolonne 1 normalerweise in herkömmlicher Weise thermisch isoliert. Die Höhe der Trennkolonne 1 ist 40 m. Der Innendurchmesser des Mantels 7 des Kolonnenkörpers 2 beträgt durchgehend 7,4 m.

In vertikaler Richtung ist die Trennkolonne 1 in drei Bereiche unterteilt: Der obere Bereich A wird als Kolonnenkopf bezeichnet. Beim Kolonnenkopf ist ein Zulauf 4 vorgesehen, über welchen eine Flüssigkeit in den Kolonnenhohlraum 3 eingeleitet werden kann. Ferner ist oben eine Abgasleitung 13 zur Entnahme des gasförmigen Gemisches ausgebildet.

Unterhalb des Kolonnenkopfes ist ein Bereich B gebildet. In diesem Bereich wird die fraktionierende Kondensation durchgeführt. Im Bereich B ist eine Entnahmeleitung 14 angeordnet, über welche Rohacrylsäure entnommen wird.

Unterhalb des Bereichs B ist der Kolonnensumpf im Bereich C gebildet. Beim Kolonnensumpf befindet sich eine Zuleitung 5 zum Einleiten des Produktgasgemisches in den Kolonnenhohlraum 3. Ferner befindet sich im Kolonnensumpf ein Ablauf 6 für die Sumpfflüssigkeit.

Im Bereich B sind im Kolonnenhohlraum 3 mehrere Böden 8 befestigt, die horizontal ausgerichtet und vertikal voneinander beabstandet sind. Die Böden 8 dienen als trennwirksame Einbauten, welche die Stofftrennung in der Trennkolonne 1 verbessern. Bei den Böden 8 handelt es sich um Regensiebböden. Es können aber auch andere der einleitend genannten Böden verwendet werden.

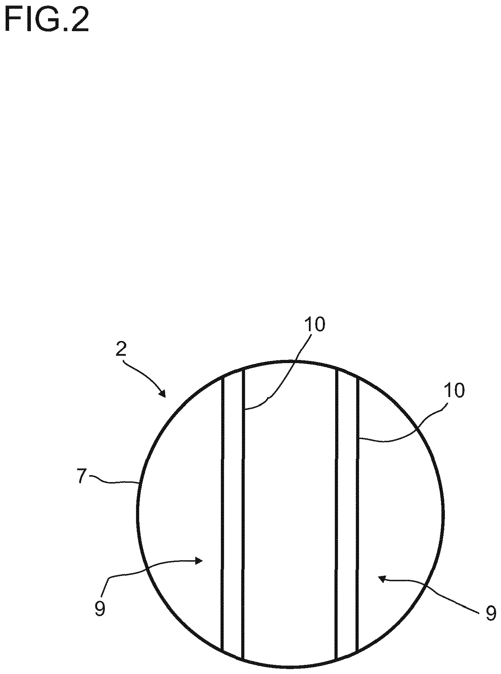

In Figur 2 ist ein horizontaler Querschnitt der Trennkolonne 1 im Bereich B zwischen zwei Böden 8 gezeigt. Im Kolonnenhohlraum 3 befindet sich innerhalb des Mantels 7 des Kolonnenkörpers 2 eine Tragekonstruktion 9, welche einen darüber angeordneten Boden 8 in vertikaler Richtung stützt. In dem in Figur 2 gezeigten Ausführungsbeispiel weist die Tragekonstruktion 9 zwei Träger 10 auf, die in horizontaler Richtung voneinander beabstandet sind. Die Träger 10 sind parallel zueinander ausgerichtet und erstrecken sich in Längsrichtung des Trägers 10 von einer Seite des Mantels 7 bis zur gegenüberliegenden Seite dieses Mantels 7. Die Träger 10 können jeweils Doppel-T-Träger sein.

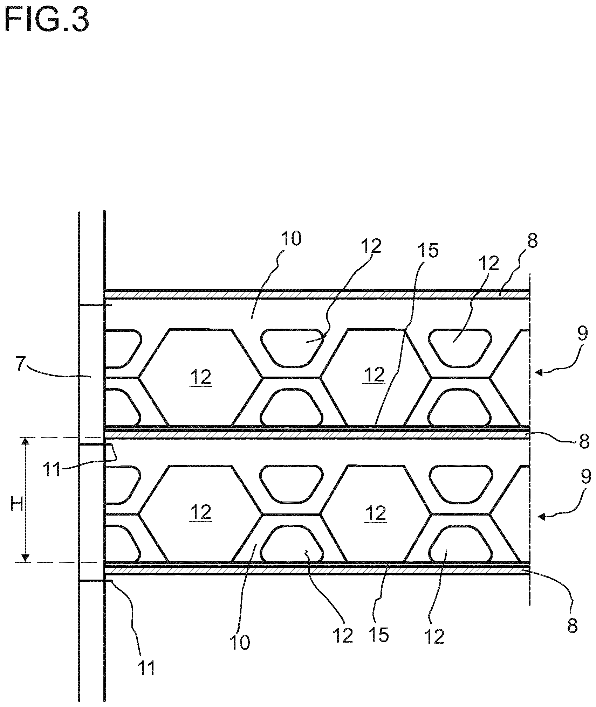

In Figur 3 ist eine Teilansicht eines vertikalen Querschnitts durch die Trennkolonne 1 gezeigt. In dieser Teilansicht sind ein Abschnitt des Mantels 7 und Abschnitte dreier Böden 8 im Bereich des Mantels 7 gezeigt. Die Böden 8 werden von Vorsprüngen 11 bei dem Mantel 7 unterstützt. Da der Durchmesser des Kolonnenkörpers 2 jedoch sehr groß ist, benötigen die Böden 8 eine weitere Unterstützung, insbesondere in der Mitte. Hierfür wird erfindungsgemäß die Tragekonstruktion 9 bereitgestellt, welche im hier beschriebenen Ausführungsbeispiel die Träger 10 umfasst, die bei dem in Figur 3 gezeigten Schnitt erkennbar sind.

Die Ausgestaltung dieser Träger 10 wird im Folgenden im Detail erläutert:
Der Träger 10 liegt auf dem unteren Boden 8 auf. Die obere Fläche des Trägers 10, der als Doppel-T-Träger ausgebildet ist, stützt die Unterseite des darüber liegenden Bodens 8 nach unten ab. Die Höhe des Trägers 10 entspricht somit dem vertikalen lichten Abstand H zwischen zwei benachbarten Böden 8. Dieser lichte Abstand H ist bei dem Ausführungsbeispiel einheitlich 400 mm.

Die Träger 10 weisen mehrere Öffnungen 12 in der vertikalen Wand auf. Die Öffnungen 12 bilden eine Wabenstruktur. Ein Teil der Öffnungen 12 ist dabei sechseckig. Die Öffnungen 12 erlauben einen horizontalen Stoffaustausch durch die von den Trägern 10 gebildete Tragekonstruktion 9.

Zumindest ein Teil der Öffnungen 12 wird dabei nach unten nur von einem Steg 15 begrenzt, der auf der Oberseite des Bodens 8 aufliegt. Die Öffnungen 12 reichen somit zum Teil sehr weit bis zur Oberseite des Bodens 8 heran. Auf diese Weise kann eine Flüssigkeit, die sich auf der Oberseite eines Bodens 8 befindet, fast ungehindert durch den Träger 10 hindurch in horizontaler Richtung fließen.

Die Wabenstruktur der Träger ist so ausgebildet, dass die Öffnungen 12 einen Anteil in einem Bereich von etwa 50% bis 60% an der vertikalen Schnittfläche des Trägers 10 einnehmen.

Wie in Figur 3 gezeigt, sind bei dieser Wabenstruktur zumindest zwei trapezförmige Öffnungen 12 vertikal beabstandet übereinander angeordnet. Diese Öffnungen 12 sind spiegelsymmetrisch bezüglich einer horizontalen Spiegelachse angeordnet, die mittig zwischen den beiden übereinander angeordneten Öffnungen 12 verläuft.

Gemäß einem anderen Ausführungsbeispiel ist die Tragekonstruktion 9 ein Gittertragwerk oder ein Fachwerk. In diesem Fall können die Öffnungen bei einem vertikalen Schnitt durch die Tragekonstruktion 9 einen Anteil von bis zu 90% an der Schnittfläche der Tragekonstruktion 9 einnehmen. Auch das Gittertragwerk oder das Fachwerk kann auf einem Boden 8 aufliegen und den darüber angeordneten Boden 8 von unten stützen. Das Gittertragwerk oder das Fachwerk kann jedoch auch unterhalb eines Bodens 8 beim Mantel 7 befestigt sein und einen Boden 8 von unten stützen, jedoch beabstandet von dem darunter liegenden Boden angeordnet sein.

Innerhalb der Öffnungen 12 oder innerhalb zumindest eines Teils der Öffnungen 12 können optional Einbauten angeordnet sein. Bei solchen Einbauten kann es sich um Packungen, insbesondere strukturierte bzw. geordnete Packungen, und/oder Schüttungen handeln.

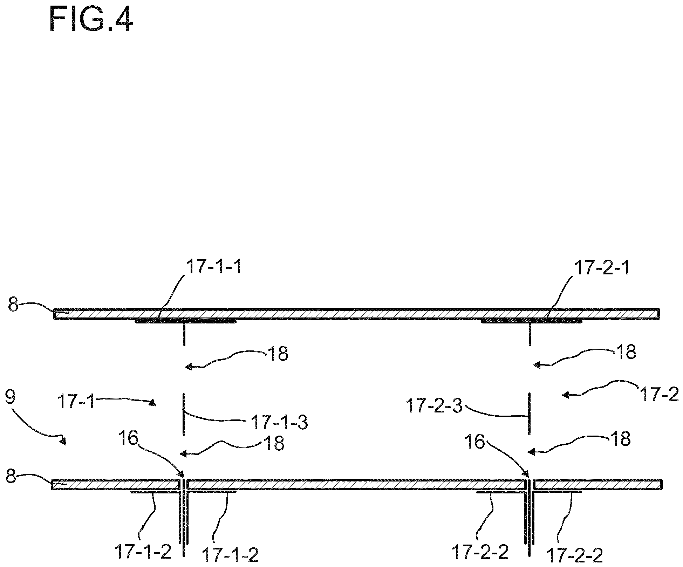

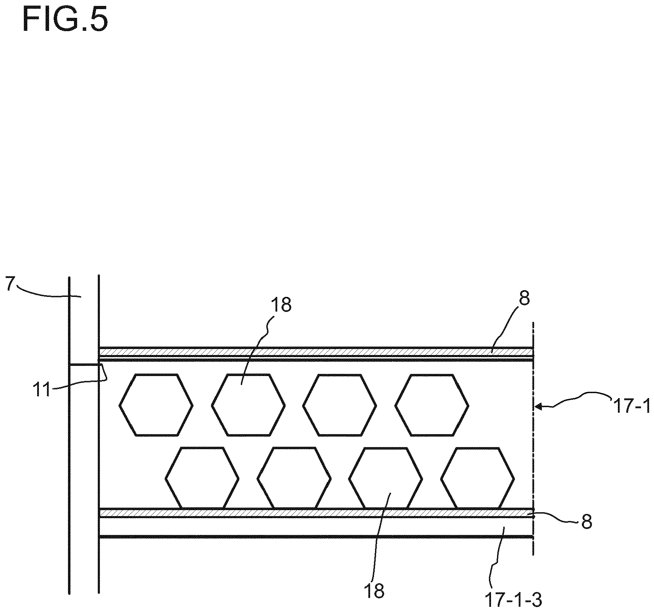

Ein noch weiteres Beispiel für die Tragekonstruktion 9 ist in den Figuren 4 und 5 gezeigt. Es werden zwei Doppel-T-Träger 17-1 und 17-2 eingesetzt, die im Bereich des Mantels 7 von den Vorsprüngen 11 unterstützt werden. Die Träger 17-1 und 17-2 weisen obere Horizontalschenkel 17-1-1 bzw. 17-2-1 auf, welche den oberen Boden 8 von unten unterstützen. Die Träger 17-1 und 17-2 weisen ferner Vertikalschenkel 17-1-3 bzw. 17-2-3 auf, die sich von den oberen Horizontalschenkeln 17-1-1 bzw. 17-2-1 jeweils nach unten erstrecken. Im unteren Boden 8 sind schlitzartige Öffnungen 16 vorgesehen, durch welche der Vertikalschenkel 17-1-3 bzw. 17-2-3 des Trägers 17-1 bzw. des Trägers 17-2 durchgeführt ist. Unterhalb des unteren Bodens 8 sind an den Vertikalschenkeln 17-1-3 bzw. 17-2-3 untere Horizontschenkel 17-1-2 bzw. 17-2-2 angebracht, die den unteren Boden 8 von unten stützen. Die Tragekonstruktion 9 stützt in diesem Fall somit zwei Böden 8.

Die unteren Öffnungen 18 sind dabei so in dem Vertikalschenkel 17-1-3 bzw. 17-2-3 angeordnet, dass die untere Kante der Öffnung 18 auf der Höhe der oberen Oberfläche des unteren Bodens 8 angeordnet ist. Auf diese Weise kann eine Flüssigkeit ungehindert auf der oberen Oberfläche des unteren Bodens 8 zirkulieren.

Die Wabenstruktur der Träger 17-1, 17-2 ist z. B. so ausgebildet, dass die Öffnungen 18 einen Anteil in einem Bereich von etwa 50 % bis 60 % an der vertikalen Schnittfläche der Träger 17-1, 17-2 einnehmen.

Wie in Figur 5 gezeigt, sind die Öffnungen 18 in zwei horizontalen Reihen angeordnet, die vertikal beabstandet voneinander sind. Dabei sind die Öffnungen 18 der zwei horizontalen Reihen horizontal versetzt zueinander angeordnet.

Die vorstehend beschriebene Tragekonstruktion 9 bildet zusammen mit den Böden 8 eine Bodeneinrichtung für eine Kolonne 1, insbesondere eine Trennkolonne 1, zur thermischen Behandlung von fluiden Gemischen.

Im Folgenden wird ein Ausführungsbeispiel des erfindungsgemäßen Verfahrens beschrieben, welches mit der vorstehend beschriebenen Trennkolonne 1 ausgeführt wird.

Bei dem Verfahren handelt es sich um ein thermisches Trennverfahren zwischen wenigstens einem in der Trennkolonne 1 aufsteigenden Gas und wenigstens einer in der Trennkolonne 1 absteigenden Flüssigkeit. Dabei enthält das aufsteigende Gas und/oder die absteigenden Flüssigkeit insbesondere (Meth)acrylmonomere.

Bei dem Trennverfahren wird eine fraktionierende Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure in einer trennwirksame Einbauten enthaltenden Trennkolonne 1 durchgeführt. Die Trennkolonne enthält von unten nach oben zunächst Dual-Flow-Böden und im Anschluss daran Querstrom-Haubenböden, die wie vorstehend beschrieben von unten gestützt werden. Ansonsten wird das Verfahren durchgeführt, wie es in den Schriften DE 19924532 A1, DE 10243625 A1 und WO 2008/090190 A1 beschrieben ist.

Unter dem Begriff "C3-Vorläufer" von Acrylsäure werden dabei solche chemischen Verbindungen zusammengefasst, die formal durch Reduktion von Acrylsäure erhältlich sind. Bekannte C3-Vorläufer von Acrylsäure sind z.B. Propan, Propen und Acrolein. Aber auch Verbindungen wie Glyzerin, Propionaldehyd oder Propionsäure sind zu diesen C₃-Vorläufern zu zählen. Von ihnen ausgehend handelt es sich bei der heterogen katalysierten Gasphasen-Partialoxidation mit molekularem Sauerstoff wenigstens teilweise um eine oxidative Dehydrierung. Bei den relevanten heterogen katalysierten Gasphasen-Partialoxidationen werden die genannten C3-Vorläufer der Acrylsäure, in der Regel mit inerten Gasen wie z.B. molekularer Stickstoff, CO, CO2, inerte Kohlenwasserstoffe und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

In typischer Weise weist das Acrylsäure enthaltende Produktgasgemisch einer heterogen katalysierten Gasphasen-Partialoxidation von C₃-Vorläufern (z.B. Propen) der Acrylsäure mit molekularem Sauerstoff an im festen Aggregatzustand befindlichen Katalysatoren, bezogen auf die Gesamtmenge der (in ihm) enthaltenen angegebenen Bestandteile, nachfolgende Gehalte auf:
1 bis 30 Gew.-% Acrylsäure,
0,05 bis 10 Gew.-% molekularer Sauerstoff,
1 bis 30 Gew.-% Wasser,
0 bis 5 Gew.-% Essigsäure,
0 bis 3 Gew.-% Propionsäure,
0 bis 1 Gew.-% Maleinsäure und/oder Maleinsäure-Anhydrid,
0 bis 2 Gew.-% Acrolein,
0 bis 1 Gew.-% Formaldehyd,
0 bis 1 Gew.-% Furfural,
0 bis 0,5 Gew.-% Benzaldehyd,
0 bis 1 Gew.-% Propen, und
als Restmenge inerte Gase wie z.B. Stickstoff, Kohlenmonoxid, Kohlendioxid, Methan und/oder Propan.

Die partielle Gasphasenoxidation selbst kann wie im Stand der Technik beschrieben durchgeführt werden. Ausgehend von Propen kann die partielle Gasphasenoxidation z.B. in zwei aufeinanderfolgenden Oxidationsstufen durchgeführt werden, wie sie z.B. in der EP 700 714 A1 und in der EP 700 893 A1 beschrieben sind. Selbstverständlich können aber auch die in der DE 19740253 A1 sowie in der DE 19740252 A1 zitierten Gasphasen-Partialoxidationen zur Anwendung kommen.

In der Regel beträgt die Temperatur des die partielle Gasphasenoxidation verlassenden Produktgasgemischs 150 bis 350°C, häufig 200 bis 300°C.

Durch direkte und/oder indirekte Kühlung wird das heiße Produktgasgemisch zweckmäßigerweise zunächst auf eine Temperatur von 100 bis 180°C abgekühlt, bevor es zum Zweck der fraktionierenden Kondensation in den Bereich C (den Sumpf) der Trennkolonne 1 geführt wird. Der in der Trennkolonne 1 herrschende Betriebsdruck beträgt in der Regel 0,5 bis 5 bar, häufig 0,5 bis 3 bar und vielfach 1 bis 2 bar.

### Bezugszeichenliste

- 1: Kolonne, Trennkolonne
- 2: Kolonnenkörper
- 3: Kolonnenhohlraum
- 4: Zulauf
- 5: Zuleitung
- 6: Ablauf
- 7: Mantel
- 8: Böden
- 9: Tragekonstruktion
- 10: Träger
- 11: Vorsprünge
- 12: Öffnungen
- 13: Abgasleitung
- 14: Entnahmeleitung
- 15: Steg
- 16: Öffnung
- 17-1,17-2: Träger
- 17-1-1, 17-1-2: Horizontalschenkel
- 17-2-1, 17-2-2: Horizontalschenkel
- 17-1-3, 17-2-3: Vertikalschenkel
- 18: Öffnungen

## Patentansprüche

1. Kolonne (1) zur thermischen Behandlung von fluiden Gemischen mit einem zylindrischen, vertikal ausgerichteten Kolonnenkörper (2), der einen Kolonnenhohlraum (3) bildet,
mehreren Böden (8), die vertikal beabstandet voneinander in dem Kolonnenhohlraum (3) montiert sind, und
einer Tragekonstruktion (9), die zumindest einen der Böden (8) in vertikaler Richtung stützt, wobei
die Tragekonstruktion (9) mehrere Öffnungen (12; 18) aufweist, die einen horizontalen Stoffaustausch durch die Tragekonstruktion erlauben, wobei
bei einem vertikalen Schnitt durch die Tragekonstruktion (9) die Öffnungen (12; 18) einen Anteil an der Schnittfläche der Tragekonstruktion (9) einnehmen, der in einem Bereich von 30% bis 90% liegt,
**dadurch gekennzeichnet, dass**
die Öffnungen (18) in zumindest zwei horizontalen Reihen angeordnet sind, die vertikal beabstandet voneinander sind, und zumindest zwei Öffnungen (12; 18) vertikal beabstandet übereinander angeordnet sind und
die Tragekonstruktion (9) auf einem Boden (8) aufliegt und einen darüber angeordneten Boden (8) von unten stützt, wobei bei zumindest einer Teilmenge der Öffnungen (12; 18) die untere Begrenzungskante der Öffnung (12; 18) von dem Boden (8), auf dem die Tragekonstruktion (9) aufliegt, oder einem Steg (15) gebildet ist, so dass diese Teilmenge der Öffnungen (12; 18) einen im Wesentlichen ungehinderten horizontalen Stoffaustausch auf der Ebene dieses Bodens (8) durch die Öffnungen (12; 18) erlaubt.

2. Kolonne (1) nach Anspruch 1, **dadurch gekennzeichnet, dass** bei einem vertikalen Schnitt durch die Tragekonstruktion (9) die Öffnungen (12; 18) einen Anteil an der Schnittfläche der Tragekonstruktion (9) einnehmen, der in einem Bereich von 40% bis 80%, insbesondere von 50% bis 60%, liegt.

3. Kolonne (1) nach einem der vorhergehenden Ansprüche, **dadurch gekennzeichnet, dass** die Tragekonstruktion (9) zumindest einen Träger (10) aufweist und dass die Öffnungen (12; 18) in einer vertikal ausgerichteten Wandfläche des Trägers (10) gebildet sind.

4. Kolonne (1) nach einem der vorhergehenden Ansprüche, **dadurch gekennzeichnet, dass** die Tragekonstruktion (9) mehrere Träger (10) aufweist und dass die Öffnungen (12; 18) in vertikal ausgerichteten Wandflächen der Träger (10) gebildet sind.

5. Kolonne (1) nach Anspruch 3 oder 4, **dadurch gekennzeichnet, dass** die Höhe des Trägers (10) oder der Träger (10) dem vertikalen Abstand benachbarter Böden (8) entspricht, so dass der Träger (10) oder die Träger (10) zwischen benachbarten Böden (8) angeordnet sind.

6. Kolonne (1) nach einem der vorhergehenden Ansprüche, **dadurch gekennzeichnet, dass** die Öffnungen (18) der zwei horizontalen Reihen horizontal versetzt zueinander angeordnet sind.

7. Kolonne (1) nach einem der vorhergehenden Ansprüche, **dadurch gekennzeichnet, dass** die Öffnungen (12; 18) eine Wabenstruktur besitzen.

8. Kolonne (1) nach einem der vorhergehenden Ansprüche, **dadurch gekennzeichnet, dass** zumindest ein Teil der Öffnungen (12; 18) sechseckig ist.

9. Kolonne (1) nach einem der vorhergehenden Ansprüche, **dadurch gekennzeichnet, dass** die horizontale Maximalerstreckung des Kolonnenkörpers (2) größer als 5 m, insbesondere größer als 7 m, ist.

10. Thermisches Trennverfahren zwischen wenigstens einem in einer Kolonne (1) nach einem der Ansprüche 1 bis 9 aufsteigenden Gas und wenigstens einer in der Kolonne (1) absteigenden Flüssigkeit.

11. Verfahren nach Anspruch 10, **dadurch gekennzeichnet, dass** das aufsteigende Gas und/oder die absteigenden Flüssigkeit (Meth)acrylmonomere enthält.

## Claims

1. A column (1) for thermal treatment of fluid mixtures, having
a cylindrical, vertical column body (2) which forms a column cavity (3),
a plurality of trays (8) mounted with mutual vertical spacing in the column cavity (3), and
a support construction (9) which supports at least one of the trays (8) in the vertical direction, where
the support construction (9) has a plurality of orifices (12; 18) which allow horizontal mass transfer through the support construction, where in a vertical section through the support construction (9) the orifices (12; 18) take up a proportion of the sectional area of the support construction (9) within a range from 30% to 90%, wherein
the orifices (18) are arranged in at least two horizontal rows with mutual vertical spacing, and at least two orifices (12; 18) are arranged one on top of another with vertical spacing and
the support construction (9) rests on a tray (8) and supports a tray (8) arranged above from beneath, where, in at least a portion of the orifices (12; 18), the lower boundary edge of the orifice (12; 18) is formed by the tray (8) on which the support construction (9) rests, or an element (15) is formed, such that this portion of the orifices (12; 18) allows essentially unhindered horizontal mass transfer along the plane of this tray (8) through the orifices (12; 18).

2. The column (1) according to claim 1, wherein, in a vertical section through the support construction (9), the orifices (12; 18) take up a proportion of the sectional area of the support construction (9) within a range from 40% to 80%, especially from 50% to 60%.

3. The column (1) according to either of the preceding claims, wherein the support construction (9) has at least one support (10) and wherein the orifices (12; 18) are formed in a vertical wall face of the support (10).

4. The column (1) according to any of the preceding claims, wherein the support construction (9) has a plurality of supports (10) and wherein the orifices (12; 18) are formed in vertical wall faces of the support (10).

5. The column (1) according to claim 3 or 4, wherein the height of the support (10) or supports (10) corresponds to the vertical spacing of adjacent trays (8), such that the support (10) or supports (10) are arranged between adjacent trays (8).

6. The column (1) according to any of the preceding claims, wherein the orifices (18) of the two horizontal rows are arranged with a horizontal offset from one another.

7. The column (1) according to any of the preceding claims, wherein the orifices (12; 18) have a honeycomb structure.

8. The column (1) according to any of the preceding claims, wherein at least some of the orifices (12; 18) are hexagonal.

9. The column (1) according to any of the preceding claims, wherein the horizontal maximum extent of the column body (2) is greater than 5 m, especially greater than 7 m.

10. A thermal separation process between at least one gas ascending within a column (1) according to any of claims 1 to 9 and at least one liquid descending within the column (1).

11. The process according to claim 10, wherein the ascending gas and/or the descending liquid comprises (meth)acrylic monomers.

## Revendications

1. Colonne (1) pour le traitement thermique de mélanges fluides, présentant
un corps de colonne cylindrique (2) orienté verticalement, qui forme une cavité de colonne (3),
plusieurs plaques (8), qui sont montées à distance verticale l'une de l'autre dans la cavité de colonne (3), et
une structure porteuse (9), qui soutient au moins une des plaques (8) en direction verticale,
dans laquelle la structure porteuse (9) présente plusieurs ouvertures (12; 18), qui permettent un échange de matières horizontal à travers la structure porteuse,
dans laquelle dans une coupe verticale à travers la structure porteuse (9), les ouvertures (12; 18) occupent une part de la face de coupe de la structure porteuse (9) qui se situe dans la plage de 30 % à 90 %,
**caractérisée en ce que**
les ouvertures (18) sont agencées en au moins deux rangées horizontales, qui sont verticalement espacées l'une de l'autre, et au moins deux ouvertures (12; 18) sont disposées à distance verticale l'une au-dessus de l'autre
et
la structure porteuse (9) repose sur une plaque (8) et soutient par-dessous une plaque (8) disposé au-dessus d'elle, dans laquelle dans au moins une quantité partielle des ouvertures (12; 18) l'arête de limitation inférieure de l'ouverture (12; 18) est formée par la plaque (8) sur laquelle la structure porteuse (9) repose, ou par une nervure (15), de telle manière que cette quantité partielle des ouvertures (12; 18) permette un échange de matières horizontal essentiellement non perturbé sur le plan de cette plaque (8) à travers les ouvertures (12; 18).

2. Colonne (1) selon la revendication 1, **caractérisée en ce que** dans une coupe verticale à travers la structure porteuse (9) les ouvertures (12; 18) occupent une part de la face de coupe de la structure porteuse (9), qui se situe dans une plage de 40 % à 80 %, en particulier de 50 % à 60 %.

3. Colonne (1) selon l'une quelconque des revendications précédentes, **caractérisée en ce que** la structure porteuse (9) présente au moins un support (10) et **en ce que** les ouvertures (12; 18) sont formées dans une face de paroi orientée verticalement du support (10).

4. Colonne (1) selon l'une quelconque des revendications précédentes, **caractérisée en ce que** la structure porteuse (9) présente plusieurs supports (10) et **en ce que** les ouvertures (12; 18) sont formées dans des faces de paroi des supports (10) orientées verticalement.

5. Colonne (1) selon une revendication 3 ou 4, **caractérisée en ce que** la hauteur du support (10) ou des supports (10) correspond à la distance verticale de plaques voisines (8), de telle manière que le support (10) ou les supports (10) soit/soient disposé (s) entre des plaques voisines (8).

6. Colonne (1) selon l'une quelconque des revendications précédentes, **caractérisée en ce que** les ouvertures (18) des deux rangées horizontales sont agencées en décalage horizontal l'une par rapport à l'autre.

7. Colonne (1) selon l'une quelconque des revendications précédentes, **caractérisée en ce que** les ouvertures (12; 18) présentent une structure en nid d'abeilles.

8. Colonne (1) selon l'une quelconque des revendications précédentes, **caractérisée en ce qu'**au moins une partie des ouvertures (12; 18) sont de forme hexagonale.

9. Colonne (1) selon l'une quelconque des revendications précédentes, **caractérisée en ce que** l'extension horizontale maximale du corps de colonne (2) est supérieure à 5 m, en particulier supérieure à 7 m.

10. Procédé de séparation thermique entre au moins un gaz ascendant dans une colonne (1) selon l'une quelconque des revendications 1 à 9 et au moins un liquide descendant dans la colonne (1).

11. Procédé selon la revendication 10, **caractérisé en ce que** le gaz ascendant et/ou le liquide descendant contient/contiennent des monomères (méth)acryliques.
